# EUROPEAN PATENT APPLICATION

(11) **EP 4 830 692 A1**
(43) Date of publication of application: **28.10.2026**
(21) Application number: 24847275.5
(22) Date of filing: 22.11.2024
(51) Int. Cl.: A61K 8/02, A61K 8/65, A61K 8/73, A61Q 19/00, A61Q 19/08

(54) **MULTILAYER-BASED COSMETIC PRODUCTS, METHOD OF PRODUCTION AND USE THEREOF**

(30) Priority: 22.12.2023 ES 202331080
(71) Applicant: Bioinicia, S.L., 46980 Paterna (Valencia) (ES); Consejo Superior De Investigaciones Científicas (CSIC), 28006 Madrid (ES)
(72) Inventor: LAGARON CABELLO, Jose Maria, 46980 Paterna Valencia (ES); PRIETO LOPEZ, Cristina, 46980 Paterna Valencia (ES); EVTOSKI, Zoran, 46980 Paterna Valencia (ES); TENO DÍAZ, Jorge, 46980 Paterna Valencia (ES); TALÓN ARGENTE, Emmanuelle, 46980 Paterna Valencia (ES); ESCRICHE BEA, Úrsula, 46980 Paterna Valencia (ES)
(74) Representative: Pons IP
(86) International application number: PCT/ES2024/070732
(87) International publication number: WO 2025/133423

(57) **Abstract**

The present invention falls within the area of polymeric materials based on ultrafine fibres applied to the cosmetics and pharmaceutical sector, the latter relating to the method and application thereof for the production of solid products with high cosmetic efficiency in multilayer format manufactured by electro-hydrodynamic, aero-hydrodynamic processing techniques or any combination of the two.

## Description

### TECHNICAL SECTOR

The present invention falls within the area of polymeric materials based on ultrafine fibres applied to the cosmetics and pharmaceutical sector, the latter relating to the method and application thereof for the production of solid products with high cosmetic efficiency in multilayer format manufactured by electro-hydrodynamic, aero-hydrodynamic processing techniques or any combination of the two.

### BACKGROUND OF THE INVENTION

Cosmetic ingredients, also called bioactive compounds, are compounds comprising biomolecules, enzymes, liposomes, anti-inflammatory agents, probiotics, prebiotics, symbiotic agents, antioxidants, cell regenerators, anti-wrinkle, anti-glare, etc.

The formulation of conventional creams faces difficulties in combining various ingredients to achieve a stable, effective and safe composition. These difficulties include ingredient compatibility, appropriate emulsification, preservation, stability, texture, appearance, the bioactivity of active ingredients, penetration and absorption into the skin, allergies and irritation, as well as regulatory compliance. The use of solid creams in micrometre or sub-micrometre fibre format can solve several of these problems.

The formation of ultrafine fibres prepared by means of electro-hydrodynamic or aero-hydrodynamic processing, e.g. electrospinning, allows products to be designed for, for example, immediate and deep absorption of cosmetic bioactives through the skin or to absorb oil from the skin more efficiently. In the electrospinning technique, the effect of the voltage on the solution causes the solvent to evaporate rapidly, causing the fibres to form immediately and act as a cosmetic ingredient per se and/or efficiently trap the cosmetic ingredient in the fibres. In this latter case, the result is an optimal encapsulation of the cosmetic ingredient within the fibre structure, as well as a reduction in the ability of its molecules to crystallise, therefore, maintaining an amorphous or quasi-amorphous state, which facilitates its dissolution, diffusion and adsorption. The micro/nano structure of these materials provides a higher surface/volume ratio and better mechanical properties compared to other techniques to generate, for example, cosmetic films that disappear on the skin. Furthermore, it allows the preparation of cosmetic formulations in a solid state, without water or oils and potentially without any preservatives or other stabilising or emulsifying additives. By generating solid creams, it is also ensured that the water activity is very low and therefore no time-consuming tests would have to be carried out to ensure the absence of microbiological contamination. These techniques increase the sustainability of the cosmetic product, by reducing weight, volume and also allow packaging to be simplified. However, polymers and active compounds may be partially retained on the substrates and therefore not optimally and completely released on the skin, requiring them to be encapsulated separately in cosmetic formulations and/or their bioactive or more bioactive form to be created in-situ during application to the skin.

The present invention aims to solve the above difficulties by formulating multilayers based on fibres processed by electro-hydrodynamic or aero-hydrodynamic techniques for their advantageous cosmetic use.

### DESCRIPTION OF THE INVENTION

The present invention proposes a cosmetic product in multilayer form (for example, to make cosmetic patches or masks), configured to be applied to the skin, capable of completely releasing bioactive agents and/or creating in-situ bioactive agents that are beneficial to the skin. The cosmetic product is manufactured using mainly electro-hydrodynamic, aero-hydrodynamic processing techniques or any combination thereof. Products can be manufactured in mono-axial form, coaxial form, by co-deposition or layer-by-layer and contain one or more bioactive agent(s) and/or precursors of bioactive agents encapsulated therein. Each layer is composed of micrometre and/or sub-micrometre scale fibres.

The bioactive agent or the precursors thereof, are encapsulated from solutions, dispersions or emulsions of the polymers and the bioactives or the precursors thereof, which will form a solid product made of ultra-fine fibres that facilitate the homogeneous and controlled release and/or the creation of the bioactives onto the skin instantly.

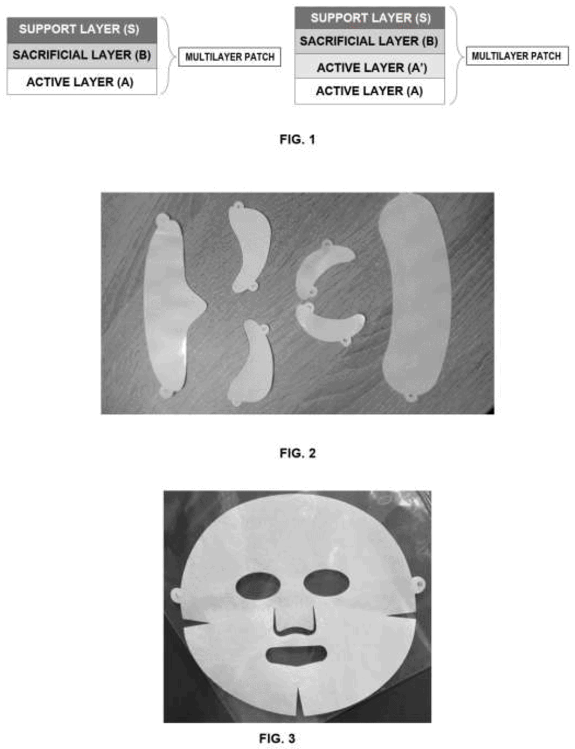

In a general manner, the products of the invention consist of between two and three layers of water-soluble polymer-based fibres, which are supported by a substrate or support layer made of electrospun fibres of any known polymer or combination of polymers, or of any woven or non-woven material (also referred to as TNT or non-woven). The support layer can also be a continuous film, which can be transparent, coloured or opaque, as well as a perforated film (see Figure 1).

A first aspect of the present invention relates to a self-adhesive cosmetic product that contains and optimally and completely releases bioactive agents and/or precursors of bioactive agents onto moistened skin, said product comprises or consists of:
- a layer of polymeric fibres (A) which is the layer that comes into contact with the skin, wherein said layer comprises at least one encapsulated bioactive agent and/or precursor of a bioactive agent, the fibres have an average diameter size between 50 nm and 5 µm, more preferably between 100 nm and 3 µm, and even more preferably between 200 nm and 1 µm, measured using scanning electron microscopy (SEM); preferably, the fibre layer has a surface density of at least 0.01 g/m², more preferably between 0.1 and 300 g/m², and even more preferably between 0.1 and 50 g/m² and wherein the polymeric fibres are formed by at least one water-soluble polymer selected from proteins or polysaccharides, as well as any mixture of the above.
- and a sacrificial layer (B) of polymeric fibres arranged immediately on layer (A), wherein said layer optionally comprises at least one encapsulated bioactive agent and/or precursor of a bioactive agent, the fibres have an average diameter size between 50 nm and 5 µm, more preferably between 100 nm and 3 µm, and even more preferably between 200 nm and 1 µm, measured using scanning electron microscopy (SEM); the fibre layer preferably has a surface density of at least 0.01 g/m², more preferably between 0.1 and 300 g/m², and even more preferably between 0.1 and 50 g/m² and wherein the polymeric fibres are formed by at least one water-soluble polymer selected from proteins or polysaccharides, as well as any mixture of the above. This layer (B) is referred to as the sacrificial layer, as most of it is not absorbed into the skin during application, but ensures that the bioactive agents and/or precursors of the bioactive agents present in layer (A) are released in the most optimal and complete way on the skin; and
- a support or substrate layer (S), characterised in that the substrate comprises a material that is insoluble in water or of lower solubility than the layers B and A, on which the layers of polymeric fibres (B) and (A) are located, thus forming a structure, from the skin outwards, A-B-S. This substrate layer (S) must preferably have a surface density of at least 0.1 g/m²; more preferably between 1 and 10,000 g/m²; even more preferably between 1 and 500 g/m².

The specific characteristics and composition of this cosmetic product allow it to adhere and dissolve quickly on moistened skin, providing an inherent effect of cosmetic firming, soothing/anti-irritation, anti-wrinkle and moisturising, which will be strengthened or complemented by encapsulated bioactive agents and/or precursors of the bioactive agent that are released and/or generate bioactive agents on the skin to generate together and in an optimal manner an enhanced anti-wrinkle, firming, soothing/anti-irritation, antibacterial, cleaning, nourishing, decongestant, antioxidant, depigmenting, regenerating, rejuvenating/anti-ageing, hydrating, emollient, illuminating, Sebum-regulating effect, etc. Additionally, by being made of very fine fibres, the large surface/volume ratio of these fibres generates a homogeneous application on the skin, copying its topology and thus facilitating its application, the immediate release and/or creation of the bioactive agents by improving their adsorption on the skin and, therefore, its effectiveness. Furthermore, by having a sacrificial layer, it is ensured that the bioactive agents and/or precursors of bioactive agents that are incorporated into the cosmetic product are deposited in large part or completely on the skin.

In a preferred embodiment, the product of the invention comprises a layer of polymeric fibres (A') between layers (A) and (B), wherein said layer comprises at least one encapsulated bioactive agent and/or precursor of the bioactive agent, the fibres have an average diameter size between 50 nm and 5 µm, more preferably between 100 nm and 3 µm, and even more preferably between 200 nm and 1 µm, measured using scanning electron microscopy (SEM); the fibre layer preferably has a surface density of at least 0.01 g/m², more preferably between 0.1 and 300 g/m², and even more preferably between 0.1 and 50 g/m² and wherein the polymeric fibres are formed by at least one water-soluble polymer selected from proteins or polysaccharides, as well as any mixture of the above.

Fibre is understood, according to the present invention, as elongated elements in which the length is greater than the width (diameter). The average diameter size of the fibres described in the present invention is measured using scanning electron microscopy (SEM).

The polysaccharides that make up the polymeric fibres of any of the layers (A), (A') or (B) may be selected independently of each layer, without limitation, from: modified pectin, alginic acid, hyaluronic acid, agar-agar, agaropectin, alpha cellulose, alpha glucan, amylopectin, amylose, beta-glucan, callose, carrageenan, cellulose, oxidised cellulose, dextran, dextrin, ficoll, fructan, fucoidan, galactogen, galactomannan, glycosaminoglycan, glucan, glycogen, glucomannan, gum arabic, cassia gum, gum sterculia, gellan gum, guar gum, gum karaya, gum tragacanth, cashew gum, xanthan gum, hemicellulose, kefiran, lentinan, levan polysaccharide, lipopolysaccharide, maltodextrin, pectin, polydextrose, pullulan, keratan sulphate, chitin, chitosan, sinistrin, xanthan, xylan, xyloglucan, fructans, microbial polysarides such as but not limited to fucopol (fucose-rich polyanionic polysaccharide), as well as any mixture of the above.

The proteins that make up the polymeric fibres of any of the layers (A), (A') or (B) may be selected independently of each layer, without limitation, from: collagen, gelatin, zein, amaranth protein, hemp protein, keratin and its hydrolysate, whey protein and its hydrolysate, pea protein and its hydrolysate, wheat protein and its hydrolysate, soya protein and its hydrolysate, elastin or any combination thereof.

In a preferred embodiment, polymeric fibres, of the layers (A), (B) or (A') independently of each other, in addition to the water-soluble polymer selected from proteins, polysaccharides, or any of the mixtures thereof, are formed by a polymer selected from PEG (polyethylene glycol) (also referred to as PEO (polyethylene oxide), hereinafter PEG referring to any PEG (low molecular weight liquid) or PEO (high molecular weight solid) polymer), polyglyceryl-4-caprate, natural deep eutectic solvents (NADES) and any combination thereof.

In a preferred embodiment, NADES, shall be selected from, but not limited to: lactic acid:sucrose, lactic acid:sucrose:water, citric acid:propylene glycol, fructose:glucose:sucrose:water, choline chloride:urea:water and any combination thereof.

In a preferred embodiment, the polymeric fibres of the layers (A), (B) or (A') independently of each other, are made up of pullulan, fructan, fucopol, cashew gum, elastin, collagen, as well as any mixture of the above. More preferably, these fibres may also be formed by other polymers selected from PEG, NADES, polyglyceryl-4-caprate and any combination thereof.

In a more preferred embodiment, the polymeric fibres of the layers (A), (B) or (A') independently of each other, are formed by at least pullulan. More preferably by pullulan or pullulan and elastin.

In a preferred embodiment, polymeric fibres, of the layers (A), (B) or (A') independently of each other, formed by a pullulan/elastin mixture where the percentage by weight of elastin in the polymeric composition is less than 10%, the remainder would be pullulan (up to 100% by weight of the polymeric mixture).

In another preferred embodiment, polymeric fibres, of the layers (A), (B) or (A') independently of each other, formed by a pullulan/PEG mixture where the percentage by weight of PEG in the polymeric composition is less than 30%, the remainder would be pullulan (up to 100% by weight of the polymeric mixture).

In another preferred embodiment, polymeric fibres, of the layers (A), (B) or (A') independently of each other, formed by a pullulan/polyglyceryl-4-caprate mixture where the percentage by weight of polyglyceryl-4-caprate in the polymeric composition is less than 6%, the remainder would be pullulan (up to 100% by weight of the polymeric mixture).

In another preferred embodiment, polymeric fibres, of the layers (A), (B) or (A') independently of each other, formed by a pullulan/collagen mixture where the percentage by weight of collagen in the polymeric composition is less than 50%, the remainder would be pullulan (up to 100% by weight of the polymeric mixture).

In another preferred embodiment, polymeric fibres, of the layers (A), (B) or (A') independently of each other, formed by a pullulan/collagen/polyglyceryl-4-caprate mixture where the percentage by weight of collagen in the polymeric composition is less than 50%, wherein the percentage by weight of polyglyceryl-4-caprate in the polymeric composition is less than 6%, the remainder would be pullulan (up to 100% by weight of the polymeric mixture).

In another preferred embodiment, polymeric fibres, of the layers (A), (B) or (A') independently of each other, formed by a pullulan/elastin/collagen mixture where the percentage by weight of elastin in the polymeric composition is less than 10%, that of collagen is less than 50%, the remainder would be pullulan (up to 100% by weight of the polymeric mixture).

Preferably, in any of the aforementioned embodiments, elastin is of plant origin (plant-based elastin), more preferably, the elastin is hydrolysed wheat protein.

Preferably, in any of the aforementioned embodiments, the collagen is of non-animal origin such as collagen of microbial origin with or without genetic modification.

Preferably, in any of the aforementioned embodiments, the PEG is PEG 400, hydrogenated castor oils PEG-10, PEG-40 and PEG-60, PEG-40 and PEG-100 stearates or PEG-20 oleate.

In a preferred embodiment, the polymeric fibres of layer (B) consist of polymer mixtures selected from the list consisting of: pullulan/elastin; pullulan/polyethylene glycol (PEG), pullulan/elastin/collagen or pullulan alone. The presence of bioactive agents in layer (B) is optional and may carry the same or different bioactive agents and/or precursors of the bioactive agent as layer (A) and/or (A').

In a more preferred embodiment, the polymeric fibres of the layer (B) are formed by a pullulan/elastin mixture or pullulan alone and do not comprise bioactive agents or precursors of bioactive agents. More preferably, the polymeric fibres of layers (A) and/or (A') are formed by pullulan/elastin.

In a preferred embodiment, the support layer (S) is formed by at least one layer of woven fibres, or woven non-woven (TNT) fibres or a continuous film of one or more hydrophilic and/or hydrophobic polymers.

In a preferred embodiment, the support layer (S) comprises or consists of a continuous film based on polyvinyl alcohol (PVOH) and/or polysaccharides, with or without strengthening additives or nanoadditives, preferably the support layer comprises or consists of starch or cellulose, for example, but not limited to, cellulose acetate; or in PCL or PHA fabrics or non-woven fabrics, as well as any combination of the above.

In an even more preferred embodiment, the support layer (S) comprises or consists of a starch-based continuous biodegradable film that is compostable and transparent with or without strengthening additives or nanoadditives, wherein the starch content is greater than 80% by weight. In an even more preferred embodiment, the starch is enriched in amylose.

In the present invention, surface density, typically expressed in g/m² for each of the layers, is calculated by weighing a sample of known dimensions. Next, said weight is divided by the surface of the sample. This process is carried out with at least 5 samples of each layer in order to thus obtain a mean surface density value for the entire layer.

In the present invention, the term "encapsulation" relates to the highly scattered and distributed incorporation of the bioactive agent in the fibres of the polymers that make up each of the layers of the patch containing same, either by forming a separate *core-shell* (core-surface) phase, or by constituting a physical mixture with the polymeric material of the fibre, including what are known as solid solutions or dispersions; the bioactive agent therefore being able to be found both inside and on the surface of said fibres, or even in the interstitial spaces between them.

In a preferred embodiment, the product of the invention consists of a layer of polymeric fibres (A) carrying bioactive agents and/or precursors of bioactive agents that will come into contact with the skin, a layer (B) immediately on layer (A) and a support layer (S). They are multi-layered structures: A-B-S.

In another preferred embodiment, the product of the invention consists of a layer of polymeric fibres (A) carrying bioactive agents and/or precursors of bioactive agents that comes into contact with the skin, a second layer of polymeric fibres (A') carrying bioactive agents and/or precursors of bioactive agents, a third layer of polymeric fibres (B) and a support layer (S) on which the previous layers are laid. They are multi-layered structures: A-A'-B-S.

In a preferred embodiment, the surface density of the layer (A) or that of the sum of the surface densities of the layers (A) and (A') is equal to or greater than the surface density of the layer (B).

In the present invention the term "bioactive" refers to, without limitation, any natural or synthetic substance, beneficial for the skin, and more preferably for use in cosmetics.

The bioactive agents that may be used in the products of the present invention are selected without limitation from any cosmetic bioactive that performs any one or a combination of the following functions:
- Anti-wrinkle: such as peptides, botox-like bioactive agents (e.g. and limitation, botulinum toxin type A, spilanthes acmella also known as biobotox, dermatorelaxants such as some polypeptides (tri, tetra, penta, octapeptides), marine derivatives such as DMAE (dimethylaminoethanol), Tsubaki oil, acetyl hexapeptide-8 (also known as argireline (Acetyl Hexapeptide-3)), or an improved version of the latter, known as SNAP-8, wakame seaweed extract, bakuchiol, etc.
- Firming agents: such as dimethylaminoethanol (DMAE), niacinamide (vitamin B3), collagen, elastin, etc.
- Soothing/Anti-irritation: such as Vitamin B12, cannabidiol (CBD), aloe vera extract, ceramides, etc.
- Antibacterial agents: such as azelaic acid, salicylic acid, malic acid, tea tree oil, etc,
- Cleaner: such as surfactants, betaine, coconut glucosides, saponins, etc.
- Nourishing: such as shea butter, almond oil, argan oil, vitamin E, etc.
- Decongestant: such as caffeine, guarana extract, ginseng extract, ginger extract, etc.
- Antioxidants: such as carotenoids, isoflavones, Vitamin C, phytosterols, etc.
- Depigmenting agents: such as glycolic acid, kojic acid, hydroxytyrosol, arbutin, etc.
- Regenerators: such as centella asiatica, epidermal growth factors, retinol, retinal, etc.
- Rejuvenating or anti-ageing: such as coenzyme Q10, jojoba oil, royal jelly, rosehip extract, etc.
- Moisturiser: such as hyaluronic acid, mucopolysaccharides, squalane, pro-vitamin B, etc.
- Emollient: such as oat extract, coconut oil, cucumber extract, sesame oil, etc.
- Illuminator: such as vitamin C, ferulic acid, lactic acid, mandelic acid, etc.
- Anti-glare: such as activated carbon, silica, clays such as bentonites, volcanic rock, etc.
- Sebo-regulator: azeloglycine, silicon, witch hazel extract, benzoyl peroxide, etc.
- Flavourings: essential oils such as lemon, tea tree, lavender, peppermint, rosemary, etc.

In another preferred embodiment, the bioactive agents and the precursors of the bioactive agents used are of synthetic origin, natural origin, biotech origin such as, for example, fermentation and/or recombinant, and they can be, but not limited to, pure, mixed, liposomed or dissolved in natural deep eutectic solvents (NADES), as well as any mixture of all the above.

In another preferred embodiment, the bioactive agents and the precursors of the bioactive agents used are derivatives or extracts of biomass, agri-food waste or by-products, or any combination of the above.

In another preferred embodiment, the bioactive agents used are derivatives from olive oil processing by-products rich in hydroxytyrosol.

In the present invention the term "precursor of the bioactive agent" refers to any substance that reacts with the moistened skin to create in situ the bioactive agent with cosmetic potency or at least two substances which being encapsulated in the polymeric fibres are released together (from the same layer) or separately (from different layers) and react with themselves to create in situ on the moistened skin the bioactive agent with cosmetic potency.

The precursors of bioactive agents which may be used in the products of the present invention are selected from, but not limited to, any biological substrate, preferably natural, for example but not limited to, enzymes, coenzymes and micro-organisms or their products such as exosomes or extracellular vesicles that transform or react with any type of molecule, preferably of natural origin, to give rise to one or more bioactive substance(s) with cosmetic functionality.

In a preferred embodiment, the precursor substances of bioactive agents encapsulated in the polymeric fibres of the layer (A) are enzymes and those encapsulated in the polymeric fibres of the layer (A') are natural extracts, or vice versa, which react upon release onto the skin and generate one or more bioactive agents with cosmetic functionality.

In a still more preferred embodiment, the precursor substance that is encapsulated in the polymeric fibres of the layer (A) is the enzyme myrosinase, and the precursor substance that is encapsulated in the polymeric fibres of the layer (A') is any cruciferous plant extract rich in the glucosinolate called glucoraphanin, or vice versa, which react on the skin to form in-situ the bioactive molecule sulforaphane with cosmetic potency. The amount of enzyme used in the product for the "in situ" creation of the bioactive molecule sulforaphane on the skin is the one that leads to the highest antioxidant capacity.

In the present invention, the term "polymer" refers to macromolecular materials of natural origin, synthetic or biotechnological in both pure ex-reactor state, and additives and post-processed materials in commercial formulas typically used by chemical industries, more commonly called plastic grades. Process additives may additionally be added to any of the polymers or plastic grades, solubility and biodegradability promoters or which confer stability, other filler-like additives, either in micro, sub-micro- or nanometric form to improve their physico-chemical properties or capacity for retention and controlled release of bioactive agents and/or the precursors of bioactive agents. Such additives can be of the chemicals type, fibres, sheets or particles.

In another preferred embodiment, any of the layers of the product of the invention may comprise aromatic substances or flavour enhancers.

The product of the invention may also comprise a pigment or ink for logo printing, pictography, either multicolour or monocolour, as a differentiating element on the sides of the product. In this case the inks or pigments used are non-toxic, are biocompatible, with good organoleptic properties and are not detrimental to the integrity of the product or the encapsulated bioactive. Any type of printing or embossing can be used for this purpose as long as it is not detrimental to the integrity of the materials of the product, as well as the encapsulated bioactive. It may also have some kind of texture.

Moreover, the product of the invention can have any size and shape or flat motif, carried out by any conventional cutting method, either manual, using a die-cutting system, or laser cutting.

As regards the manufacture of the fibres that make up the layers of the product of the invention, these are preferably carried out by means of any of the known electro-hydrodynamic and aero-hydrodynamic techniques for obtaining fibres, more preferably using electrospinning, direct beam printing using electro-hydrodynamic processing *(electrohydrodynamic direct writin*g), melt electrospinning, solution blow spinning*,* as well as by means of any combination and/or variant thereof. Nevertheless, any other method for obtaining fibres may also be used, such as centrifugal jet spinning or the combination of this and those previously mentioned. Electro-hydrodynamic and aero-hydrodynamic techniques are based on the formation of polymeric micro- or submicrofibres at room temperature or lower, from a solution, polymer suspension or emulsion to which an electric field or gas pressure is applied. The fact that it is processed in liquid form provides great versatility, as it allows the incorporation of several substances at the same time. At the same time, the fact that its processability is at room temperature avoids certain problems such as degradation of the bioactive agent or precursors of the bioactive agent.

In a preferred embodiment, the product of the invention is manufactured by means of electrospinning. In an even more preferred embodiment, they are carried out by electrospinning using multi-output or multi-emitter injectors, whether made of needles or similar or made of porous materials. The advantage of these injectors over the so-called free-surface injectors is that they do not have a controlled output, also called *needleless electrospinning* or *free surface electrospinning*, the greater control of fibre diameter and fibre diameter dispersion and also of homogeneity throughout the thickness. Control of fibre diameter dispersion facilitates reproducibility in release kinetics and thus cosmetic certification.

In a preferred embodiment the variation in fibre diameter is less than 35%, in other words, that the fibre diameter variation is less than ±17.5% above the average value. This value is measured by scanning electron microscopy (SEM).

In another preferred embodiment, fibre diameter variation for a given system with a multi-outlet injector is at least 15% less than that which would occur with uncontrolled outlet injectors.

In another even more preferred embodiment, fibre diameter variation for a given system with a multi-outlet injector is at least 5% less than that which would occur with uncontrolled outlet injectors.

With these techniques and the polymers mentioned above, in the present invention, the bioactive agent(s) and/or the precursor(s) of the bioactive agent(s) are encapsulated in such a way that the release can also be sustained. To carry out this encapsulation, techniques are used which include, without limitation: core-shell technology, co-deposition, surface modification electrospinning, electrospinning side-by-side, to generate Janus-type structures, direct mixing, emulsion techniques, pre-encapsulation in particles, layer by layer deposition, etc.

In the present invention, core-shell technology is used in the case of electrospinning and solution blow spinning, using a concentric nozzle through which one or more bioactive agent(s) and/or one or more precursor(s) of the bioactive agent(s) in solution are delivered through the inner tube with or without encapsulating polymers, while the same or other encapsulating polymers and optionally the same and/or other bioactive agents and/or precursors of bioactive agents, are passed through the outer tube, or vice versa. The use of nozzles with more than two concentric tubes (triaxial or similar), can lead to further combinations of bioactives, precursors and polymers. Either way, this technology may result in tubular fibres that may contain different bioactive agents and/or different precursors of bioactive agents in the inner layer and in the outer layer of the coaxial or multi-axial fibre. It also allows, depending on the design, fibres to be made with controlled release profiles of the bioactive agents or precursors of the bioactive agents.

In the present invention, co-deposition consists of a deposition method in which two injectors simultaneously deposit identical or different fibres, for example, on the one hand, a polymeric solution containing one or more bioactive agents and/or one or more precursors of bioactive agents and on the other hand, the same or another polymeric solution with the same or other bioactive agents and/or precursors of bioactive agents.

This technique is used to separate bioactive agents or precursors of non-compatible or potentially reactive bioactive agents and/or to control the release thereof. It is also used by depositing two solutions having different types of polymers, thus generating different fibre sizes and morphologies within the same membrane, and thus a different release profile of the bioactive agent(s) and precursors. Co-deposition can also be performed with fibres, particles and/or mixtures of the two. Therefore, co-deposition or simultaneous electro-spinning allows the combination of various compositions and properties within the same layer.

In the present invention, the direct mixture can be, without limitation, of the encapsulated perfume and the encapsulating agent or of a suspension of particles containing the preencapsulated bioactive agent or precursor and the encapsulating agent, using monoaxial electrospinning, giving rise to cylindrical fibres in which the bioactive agent or precursor is embedded and dispersed within the fibre. Said mixture can be a homogeneous solution or a heterogeneous suspension.

In the present invention, the emulsion techniques refer to any emulsion of, without limitation, solvents or components, which give rise to encapsulation with several phases and which are processed by the processes known as electrohydrodynamic or aero-hydrodynamic emulsion processing. An emulsion is a dispersion of a liquid (dispersed phase) in the form of very small droplets within another liquid (continuous phase) with which it is generally immiscible. Emulsions can be direct, inverse or multiple. Direct emulsions are those in which the dispersed phase is a lipophilic substance and the continuous phase is hydrophilic. These emulsions are often called L/H or O/W. Inverse emulsions, on the other hand, are those in which the dispersed phase is a hydrophilic substance and the continuous phase is lipophilic. These emulsions are often called the abbreviation H/L or W/O. They can also be O/O emulsions, with two immiscible organic phases. Multiple emulsions are those in which, for example, the dispersed phase contains an inverse emulsion and the continuous phase is an aqueous liquid. Multiple emulsions can be H/L/H or W/O/W or O/W/O. Emulsions can also be formulated by means of the so-called Pickering emulsions that use particles to separate and stabilise the phases and by means of any other type of emulsion technology. In this way, the bioactive agent and/or the precursor of the bioactive agent are encapsulated within the fibres in one of the phases, typically in the organic phase, and the release can also occur in a controlled manner.

In the present invention, pre-incorporation technologies can also be used for functionalisation, stabilisation or separation of active agents and/or precursors of bioactive agents within liposomes, nanoliposomes, NADES, solid lipid particles and nanoparticles, or inclusion complexes, for example, in cyclodextrins. Additionally, pre-encapsulation may also use any other encapsulation method that produces particles such as, and not limited to, *electrospray,* air-assisted electrospray (EAPG), coacervation, emulsion-evaporation/emulsion-extraction, hot melt, interfacial polycondensation, complexing, gelling, fluidised bed, atomisation, lyophilisation, extrusion*, electrostatic droplet generation,* supercritical fluids, *TROMS,* etc., and mixtures thereof. These particles with bioactive and/or precursors are typically added, in the case of direct mixing, to a solution of the selected biopolymer(s), such that, after any of the aforementioned processes, fibres with particles inside are obtained. In this case, the controlled release method of the encapsulate is carried out by dissolution, diffusion or opening of the particles and fibres, or even by a combination of several of the above mechanisms.

In the present invention, the layer-by-layer deposition method consists of the use of a process in which the layers are deposited sequentially. In this regard, initially, the layer (B) is deposited on the support layer (S) until the desired surface density is reached and then the following layers (A') and (A) are deposited, obtaining a multilayer product *in situ*.

Therefore, another aspect of the invention relates to a method for producing the product described in the present invention, wherein said method is based on the electro-spinning technique comprising:
preparing each fibre layer from a solution, suspension or emulsion of the polymer or polymer mixture to be formed into fibres wherein the polymer or polymer mixture is in a concentration of between 0.1 and 60% by weight; the emitter voltage used is between 0.01 and 500 kV and a collector voltage is between 0 kV and -500 kV, with a flow rate of 0.0001 to 50,000 ml/h, at a temperature of between 1°C and 100°C, more preferably between 20 and 40°C; and a relative humidity between 10 and 60% and more preferably between 15 and 40%.

When the layers carry bioactive agents and/or precursors of bioactive agents, these are added to the solution, suspension or emulsion of the polymer or polymer mixture. Preferably, the bioactive agent(s) and/or precursor(s) of bioactive agent(s) are in a total concentration between 0,0001 and 70% by weight in said solution, suspension or emulsion.

Preferably, a solution of the polymer or polymer mixture is prepared by choosing a solvent in which the polymers are soluble at the temperature at which the process is carried out. The preferred solvents are water and alcohols, such as methane, ethanol, isopropanol, butanol and trifluoroethanol, or any mixture thereof, more preferably, the solvent used is water.

When the polymer forming the fibres is not chemically compatible with the bioactive agent or precursor to be encapsulated (there is no physico-chemical interaction between them) or they have a low or very low solubility in the solvent(s) of the biopolymer required to carry out the manufacturing process of the product, then any known emulsion route can be used to encapsulate the bioactive agent(s) and/or the precursor(s) of the active agent(s), rather than the direct dissolution or suspension thereof. Solvents are preferably water or alcohols (such as methane), ethanol, isopropanol, butanol and trifluoroethanol), or any mixture of the above.

In a preferred embodiment, the layers of polymeric fibres are prepared layer by layer in the following order: first the layer (B) on top of the substrate layer (S), then the layer (A') and finally the layer (A). The substrate (S) being as defined above. Alternatively, it is also possible to obtain the various layers separately, and they are laminated to obtain the product of the invention, A-B-S or A-A'-B-S, using any known lamination method.

The present invention also relates to the cosmetic use of the cosmetic product of the present invention based on water-soluble polymers that in and of themselves already produce an inherently beneficial cosmetic effect on the skin, as well as for the complete or near-complete delivery of one or more bioactive agent(s) and/or precursor(s) of bioactive agent(s) present in the product, on the skin.

The expression "inherently beneficial cosmetic effect" implies that the product itself while only based on water-soluble polymers, is able to produce a beneficial cosmetic effect on the skin. The "beneficial cosmetic effect" refers to a smoothing, firming, soothing, anti-irritation, anti-wrinkle and moisturising effect.

Therefore, another aspect of the present invention relates to the cosmetic use of the product defined in the present invention as a patch or mask to produce a beneficial cosmetic effect on moistened skin and for the controlled release of one or more bioactive agents and/or one or more precursors of bioactive agents in the moistened skin.

Another aspect of the invention relates to a cosmetic method of using the product described in the present invention for its preferred application on the skin: wherein the method comprises: (i) wetting or spraying the skin with any type of water, including thermal waters, at any temperature or with a cosmetic mist, (ii) the product is then applied on the skin by bringing the layer (A) in contact with the skin, (iii) the product is then left for a few seconds, at least 3 seconds, on the skin, preferably between 3 and 5 seconds, and (iv) finally, the layer (S) is carefully removed and discarded.

The term "moistened skin" refers in the present invention to skin treated according to step (i) of the above described method. In a preferred embodiment, the skin wetting is performed with a cosmetic mist.

Preferably during the application method, the layers (A) and (A') will be mostly or completely absorbed on the skin, releasing the bioactive agents and/or the precursors of the bioactive agents and the sacrificial layer (B) will be mostly retained on the substrate (S) and the layer (B) could be removed together with the layer (S).

Another aspect of the invention relates to a kit comprising the cosmetic product described above and a water-based spray container (also called a mist) with or without other cosmetic, balsamic and/or odoriferous products. The spraying device allows the skin to be moistened before application of the cosmetic product so that the product adheres to the skin easily and dissolves on the surface thereof.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

**Fig. 1****.** shows the different multilayer configurations of the product of the invention.
**Fig. 2****.** shows patches of the cosmetic product described in example 1.
**Fig. 3****.** shows a mask of the cosmetic product described in example 1.
**Fig. 4****.** shows the viability of the keratinocyte cell line (HaCaT) after incubation for 24 hours according to the resazurin assay in contact with the patch of example 1.
**Fig. 5****.** shows the quantification of reactive oxygen species (ROS) as arbitrary fluorescence units (AFU) per microgram of total proteins of the keratinocyte cell line (HaCaT) after 24 hours in contact with the patch of example 1.

### EXAMPLES

Next, the invention will be illustrated by means of assays carried out by the inventors that demonstrate the effectiveness of the product of the invention.

### Example 1: Multilayer cosmetic product with pullulan/elastin (B), pullulan/elastin/hydroxytyrosol-rich extract (A) on a starch-based substrate film (S).

On the starch-based film (100 µm thick) placed on a rotating collector (200 rpm) the hydrophilic fibres with encapsulated bioactive agent were deposited. Said manufacture was carried out by electrospinning in a linear multi-outlet injector with 5 needles, at a temperature of 30°C and a relative humidity of 30%.

To do so, the starting point was a solution of pullulan at 14.55% by weight (wt.%), 0.45% hydrolysed wheat protein (plant-based elastin) in deionised water, to generate a first layer (B) with a surface density of 2 g/m².An emitter voltage of 28kV was used to produce the fibre mesh, as well as a collector voltage of -25V, and a flow rate of 5 ml/h.

On the previous layer, a second layer of fibres (A) was deposited starting from a pullulan solution at 14.55% by weight (wt.%), 0.45% hydrolysed wheat protein (plant-based elastin), and 0.2688% of an extract rich in hydroxytyrosol, in deionised water. In this polymeric solution, the polymer:bioactive ratio was 98.24:1.76. To do so, an emitter voltage of 28 kV was used, as well as a collector voltage of -25V, and a flow rate of 5 ml/h, with a surface density of 3 g/m².

The product in this example was cut into different shapes to be applied on different parts of the skin of the face as shown in figures 2 and 3. HPLC confirmed that after applying the product on the forehead skin of three volunteers for three seconds, moistened with a mist, less than 10% of the hydroxytyrol in the patch was retained on the substrate (S).

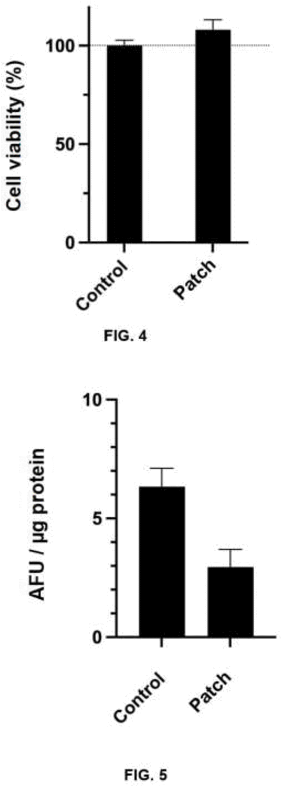

Figure 4 indicates by *in vitro* assay that the cosmetic product is not cytotoxic to skin, as the viability of keratinocytes is not reduced after 24 hours in contact with the dissolved material of the cosmetic patch. Figure 5 indicates that the cosmetic product has a strong ability to reduce the production of reactive oxygen species and thus has an anti-ageing effect.

### Example 2: Multilayer cosmetic product with pullulan (B), pullulan/elastin/hydroxytyrosol- and hyaluronic acid-rich extract (A) on a starch-based substrate film (S).

On the starch film (100 µm thick) placed on a rotating collector (200 rpm) the hydrophilic fibres with encapsulated bioactive agent were deposited. Said manufacture was carried out by electrospinning in a linear multi-outlet injector with 5 needles, at a temperature of 30°C and a relative humidity of 30%.

To do so, the starting point was a solution of pullulan at 15% by weight (wt.%) in deionised water, to generate a first layer B with a surface density of 2 g/m². An emitter voltage of 28kV was used to produce the fibre mesh, as well as a collector voltage of - 25V, and a flow rate of 5 ml/h.

On top of the previous layer another layer of fibres was deposited starting from a solution of pullulan at 14.55% by weight (wt.%), 0.45% hydrolysed wheat protein (plant-based elastin), 0.271% of an extract rich in hydroxytyrosol and 0.155% of hyaluronic acid with a molecular weight of 5 kDa, in deionised water. In this solution, the polymer:bioactive ratio was 97.24:2.76. To produce this second layer of fibres (A), an emitter voltage of 28 kV was used, as well as a collector voltage of -25V, and a flow rate of 5 ml/h, with a surface density of 3 g/m².

### Example 3: Multilayer cosmetic product with pullulan/elastin (B), pullulan/elastin/broccoli extract (A') and pullulan/elastin/myrosinase (A) on a starch-based substrate film (S).

On the starch film (100 µm thick) placed on a rotating collector (200 rpm) the hydrophilic fibres with encapsulated bioactive agent were deposited. Said manufacture was carried out by electrospinning in a linear multi-outlet injector with 5 needles, at a temperature of 30°C and a relative humidity of 30%.

To do so, the starting point was a solution of pullulan at 14.55% by weight (wt.%), 0.45% hydrolysed wheat protein (plant-based elastin) in deionised water, to generate a first layer B with a surface density of 3 g/m². An emitter voltage of 28kV was used to produce the fibre mesh, as well as a collector voltage of -25V, and a flow rate of 5 ml/h.

On top of the previous layer another layer of fibres (A') was deposited starting from a solution of pullulan at 14.55% by weight (wt.%), 0.45% hydrolysed wheat protein (plant-based elastin) and 3.52% broccoli extract, in deionised water. In this solution the polymer:bioactive ratio was 96.47:3.53. To produce this second layer of fibres (A'), an emitter voltage of 28 kV was used, as well as a collector voltage of -25V, and a flow rate of 5 ml/h, with a surface density of 3.2 g/m².

Another layer of fibres (A) was deposited on the previous layer, the starting point was a solution of pullulan at 14.55% by weight (wt.%), 0.45% hydrolysed wheat protein (plant-based elastin), and 0.5% of the enzyme myrosinase, in deionised water. In this solution, the ratio of polymer:bioactive was 99.994:0.006. To produce this second layer of fibres (A), an emitter voltage of 28 kV was used, as well as a collector voltage of -25V, and a flow rate of 5 ml/h, with a surface density of 0.8 g/m². The amount of enzyme used for the "in situ" creation of the bioactive antioxidant molecule on the skin was optimised to obtain the highest antioxidant value measured by the *in vitro* method of DPPH.

In this type of patch, the water-soluble layers containing the two precursors, extract and enzyme, are solubilised in the moistened skin to react and create the bioactive molecule sulforaphane in situ.

## Claims

1. A self-adhesive cosmetic product to be applied to moistened skin, wherein the product comprises:
- a layer of polymeric fibres (A) which is the layer in contact with the skin, wherein the fibres have an average diameter size between 50 nm and 5 µm; and are formed by at least one water-soluble polymer selected from polysaccharides, proteins, and any of the mixtures thereof and, wherein it additionally comprises at least one bioactive agent and/or at least one precursor of a bioactive agent encapsulated in the layer (A),
- a sacrificial layer of polymeric fibres (B), wherein the fibres have an average diameter size between 50 nm and 5 µm; and are formed by at least one water-soluble polymer selected from polysaccharides, proteins, and any of the mixtures thereof, and wherein it optionally comprises at least one bioactive agent and/or at least one precursor of a bioactive agent encapsulated in the layer (B), and
- a support or substrate layer (S), **characterised in that** the substrate comprises a material that is insoluble in water or of lower solubility than the layers (B) and (A), and on which the layers of polymeric fibres (B) and (A) are located, thus forming a structure, from the skin outwards, (A)-(B)-(S).

2. The cosmetic product, according to claim 1, wherein the product additionally comprises a layer of polymeric fibres (A'), arranged between layers (A) and (B), wherein the fibres have an average diameter size between 50 nm and 5 µm; and are formed by at least one water-soluble polymer selected from among, polysaccharides, proteins, and any of the mixtures thereof, and that additionally comprises at least one bioactive agent and/or at least one precursor of a bioactive agent encapsulated in the layer (A').

3. The cosmetic product, according to claim 1 or 2, wherein the polymeric fibres, of the layers (A), (B) or (A') independently of each other, are made up of pullulan, fructan, fucopol, cashew gum, elastin, collagen or any of the combinations thereof.

4. The cosmetic product, according to any of claims 1 to 3, wherein the polymeric fibres, of the layers (A), (B) or (A') independently of each other, are additionally formed by a polymer selected from PEG, NADES, polyglyceryl-4-caprate and any combination thereof.

5. The cosmetic product according to any of claims 1 to 4, wherein the polymeric fibres of at least one of the layers (A), (A') and (B) are formed by a pullulan/elastin mixture where the percentage by weight of elastin in the polymeric composition is less than 10% and the remainder is pullulan.

6. The cosmetic product according to any of claims 1 to 4, wherein the polymeric fibres of at least one of the layers (A), (A') and (B) are formed by a pullulan/PEG mixture where the percentage by weight of PEG in the polymeric composition is less than 30% and the rest is pullulan.

7. The cosmetic product according to any of claims 1 to 4, wherein the polymeric fibres of at least one of the layers (A), (A') and (B) are formed by a pullulan/collagen mixture where the percentage by weight of collagen in the polymeric composition is less than 50% and the remainder is pullulan.

8. The cosmetic product according to any of claims 1 to 4, wherein the polymeric fibres of at least one of the layers (A), (A') and (B) are formed by a pullulan/collagen/polyglyceryl-4-caprate mixture where the percentage by weight of collagen in the polymeric composition is less than 50%, where the percentage by weight of polyglyceryl-4-caprate in the polymeric composition is less than 6% and the remainder is pullulan.

9. The cosmetic product according to any of claims 1 to 4, wherein the polymeric fibres of at least one of the layers (A), (A') and (B) are formed by a pullulan/elastin/collagen mixture and the percentage by weight of elastin in the polymeric composition is less than 10%, that of collagen is less than 50% and the rest is pullulan.

10. The cosmetic product according to any of claims 1 to 4, wherein the polymeric fibres of the layer (B) are formed by a pullulan/elastin mixture or pullulan alone and do not comprise bioactive agents or precursors of bioactive agents.

11. The cosmetic product, according to any of claims 3, 5, 9 and 10, wherein the elastin is a plant-based elastin.

12. The cosmetic product, according to claim 11 wherein the plant-based elastin is hydrolysed wheat protein.

13. The cosmetic product, according to any of claims 1 to 12, wherein the support or substrate layer (S) consists of a continuous biodegradable film that is compostable and transparent, comprising more than 80% by weight of starch.

14. The cosmetic product, according to any of claims 1 to 13, wherein the fibres of any of the layers are obtained by electro-hydrodynamic, aero-hydrodynamic techniques or a combination thereof.

15. The cosmetic product according to claim 14, wherein the fibres of any of the layers are obtained by electro-spinning*.*

16. The cosmetic product, according to any of claims 1 to 15, wherein the surface density of the layer (A) or that of the sum of the surface densities of the layers (A) and (A') is equal to or greater than the surface density of the layer (B).

17. The cosmetic product, according to any of claims 2 to 16, wherein the precursor substance of the bioactive agent comprising the polymeric fibres of the layer (A) is the enzyme myrosinase, and the one comprising the polymeric fibres of the layer (A') is a cruciferous extract containing glucoraphanin, or vice versa.

18. A method of production of the cosmetic product defined in any of claims 1 to 17, wherein said method is based on the electrospinning technique comprising:
preparing each fibre layer from a solution, suspension or emulsion of the water-soluble polymer or polymer mixture to be formed into fibres where the polymer or polymer mixture is in a concentration of between 0.1 and 60 % by weight; the emitter voltage used is between 0.01 and 500 kV and a collector voltage is between 0 kV and -500 kV, with a flow rate of 0.0001 to 50,000 ml/h, at a temperature between 1 and 100°C and a relative humidity between 10 and 60%; and
adding to the solution, suspension or emulsion of the layer (A) at least one bioactive agent, at least one precursor of bioactive agent or any of the combinations thereof at a total concentration between 0.0001 and 70% by weight in said solution, suspension or emulsion.

19. The method, according to claim 18, wherein at least one bioactive agent, at least one precursor of bioactive agent or any of the combinations thereof is added to the solution, suspension or emulsion of layer (A') and/or (B) at a total concentration between 0.0001 and 70% by weight in said solution, suspension or emulsion.

20. The method, according to claim 18 or 19, wherein the fibre layers (B), (A') and (A) are prepared layer by layer on a substrate layer (S).

21. The method, according to any of claims 18 to 20, wherein controlled outlet, multi-outlet or multi-transmitter injectors are used.

22. A cosmetic use of the product defined in any one of claims 1 to 17 as a patch or mask to produce a beneficial cosmetic effect on moistened skin.

23. The cosmetic use of the product defined in any one of claims 1 to 17, as a patch or mask for the controlled release of one or more bioactive agent(s) and/or one or more precursors of bioactive agents on the moistened skin.

24. A cosmetic method for use of the product defined in any of claims 1 to 17, which comprises: (i) moistening or spraying the skin with water, (ii) then applying the product on the skin (i) by bringing the layer (A) in contact with the skin, (iii) then leaving the product for at least 3 seconds on the skin, preferably between 3 and 5 seconds, and (iv) removing the layer (S) and discarding it.

25. A kit comprising a cosmetic product, according to any of claims 1 to 17, and a spray container comprising water or water with at least one cosmetic, balsamic, odoriferous product or any of the combinations thereof.
